# EUROPEAN PATENT APPLICATION

(11) **EP 4 700 048 A1**
(43) Date of publication of application: **25.02.2026**
(21) Application number: 25192864.4
(22) Date of filing: 30.07.2025
(51) Int. Cl.: C08B 37/08, C08J 5/18, C09D 105/04, C09D 105/08, C09D 199/00, C08B 37/00, C08L 79/02

(54) **THIN FILM CONTAINING A PLANT-BASED AGENT AND METHODS OF MANUFACTURING THEREOF**

(30) Priority: 02.08.2024 PL 44944124
(71) Applicant: Politechnika Wroclawska, 50-370 Wroclaw (PL)
(72) Inventor: Tsirigotios-Maniecka, Marta, 51-313 Wroclaw (PL); Pawlaczyk-Graja, Izabela, 55-002 Dobrzykowice (PL)
(74) Representative: Czarnik, Maciej

(57) **Abstract**

The invention discloses a thin film comprosing a plant-based agent in the form of polysaccharide macromolecules with a molecular weight > 4 kDa, obtained from wild strawberry leaves with a surface charge density expressed as a zeta potential in the range from -50 mV to -15 mV, and consists of a first layer in the form of polyethylene diamine, a second layer in the form of calcium alginate, a third layer in the form of chitosan, and a fourth layer in the form of a plant-based agent. The invention also discloses a method for obtaining a thin film.

## Description

The subject matter of the invention relates to a thin film containing a plant-based agent and methods of manufacturing the same, applicable in medical engineering, preferably in biomedical engineering, as a biomaterial exhibiting anticoagulant, antioxidant, and gamma radiation protective properties.

A plant-based agent is known from Polish patent description No. Pat.216635, intended for use as a medicinal component, antioxidant and/or preservative. The agent contains, as an active substance, a polyphenol-polysaccharide mixture comprising macromolecules characterized by a molecular weight of ≥ 5 kDa, isolated from plants of the Rosaceae family, such as meadowsweet (*Filipendula ulmaria*), bird cherry (*Cerasus padus*), wild strawberry *(Fragaria vesca),* great burnet (*Sanguisorba officinalis*), midland hawthorn (*Crataegus laevigata*), European blackberry (*Rubus plicatus*), common agrimony (*Agrimonia eupatoria*); and from plants of the Asteraceae family, including yarrow (*Achillea millefolium*), purple coneflower (*Echinacea purpurea*), Canadian fleabane (*Conyza canadensis*), mountain arnica (*Amica montana*), European goldenrod (*Solidago virgaurea*), chamomile (*Matricaria chamomilla*), coltsfoot (*Tussilago farfara*)*.* The subject matter of the above invention also concerns a method of manufacturing a plant-based antioxidant agent, wherein the aforementioned plant raw material is subjected to extraction with water and/or lower alcohols or an aqueous alkaline solution and/or an alkaline salt solution, followed by neutralization to pH = 5.5 - 7.5 using aqueous solutions of inorganic acids. In a second stage, substances having a molecular weight of ≥ 5 kDa are isolated from the obtained extracts. For this purpose, the plant extract dissolved in water and/or in an alkaline solution is subjected to filtration through a barrier impermeable to components of the plant extract with a molecular weight of ≥ 5 kDa, and subsequently dried to a powdered form.

Polysaccharide films and coatings for surface modification are defined as any material used for coating a product in order to impart new physical, chemical, biological, or mechanical properties, to extend its shelf life by protecting it against physical, chemical, and microbiological changes, or to improve the quality of the product. Films or coatings for surface modification, suitable for industrial applications, may be homogeneous or heterogeneous, single-layered or multi-layered. The objective of producing films and coatings for surface modification is to obtain stable and thin, yet multifunctional materials.

Extensive research on polysaccharide films has demonstrated that pectins and pectin-like compounds are highly valuable in this context (Pasini Cabello et al., 2015). Pectins are macromolecular substances constituting up to 30% of the dry mass of plants and represent the most abundant compounds in the cell walls of higher plant tissues. These compounds differ not only in monosaccharide composition but also in chain structure, molecular weight, and types of glycosidic bonds. They are mixtures of natural, hydrophilic, anionic heteropolysaccharides composed of residues of D-galacturonic acid (including partially methylated and acetylated forms) and neutral saccharides, e.g., rhamnose, arabinose, primarily linked by α-(1,4)-glycosidic bonds. Pectins are most often linear polymers, although branched pectins are also known, with side chains consisting of monosaccharide subunits lacking electrically charged functional groups. The degree of esterification or the number of methoxyl groups attached to galacturonic acid residues frequently serves as a classification criterion for pectins. In the structure of certain pectins, e.g., those isolated from sugar beet leaves and spinach leaves, other functional units covalently bound to saccharide residues may also be present, such as ferulic acid, proteins, and the like. These structural differences translate into distinct physicochemical properties. Fruit-derived pectins are most commonly employed as multifunctional viscosity modifiers, thickening agents, coating agents, gelling agents, emulsifiers, or stabilizers in food and cosmetic products, pharmaceutical formulations, and even as biomaterials for tissue engineering and wound dressings, owing to their very low toxicity, high biocompatibility, and biodegradability.

In the production of films and coatings based on pectins, the phenomenon of inter- and intramolecular association or cross-linking of polymer chains is utilized, forming a coating matrix that entraps and immobilizes the solvent. The degree of cohesion depends on the chemical structure of the polymer (chain conformation, type and distribution of monosaccharides in the chain backbone, glycosidic bonds between monosaccharides, number and type of branching), the molecular weight of the polysaccharide, the solvent used (e.g., ionic strength, pH), temperature, the presence of other chemical compounds, and micro- and/or nanoparticles. Unfortunately, the use of commercial fruit pectins in this regard is limited due to structural variability of these compounds, which consequently results in diverse physicochemical properties of pectin films (Pasini Cabello et al., 2015). Moreover, pectin films are poor moisture barriers as they rapidly hydrate, swell, and subsequently erode in water environment (Al-Tayyar et al., 2020). These characteristics reduce their suitability for effectively controlling the release of active substances under specific conditions. Nevertheless, pectin-based films and coatings exhibit excellent mechanical properties, and their sensory, nutritional, and functional characteristics can be modified by adding other biopolymers, cross-linking agents, plasticizers, emulsifiers, or surfactants. New non-covalent, covalent, ionic, hydrogen, or hydrophobic bonds form between pectins and such additives. For example, the mechanical properties of thin films composed of pectin-chitosan electrostatic complexes depend on the molecular weight of the polymers, the degree of chitosan deacetylation, and the pH value of the environment - films swell and may dissolve in acidic conditions, while their solubility decreases at pH 6 and near-neutral pH. The use of cross-linking agents in the formation of pectin films and coatings improves their water resistance, cohesion, rigidity, optical properties, and mechanical strength (Zink et al., 2016). Meanwhile, surfactants, by reducing surface tension, enhance the wettability of pectin films and coatings and facilitate the adhesion of the coating material to the substrate (Rodriguez et al., 2006). Pectin films can also serve as carriers for antimicrobial agents, antioxidants, nutraceuticals, aromas, dyes, and the like, which modify their functional properties (Chen et al., 2020).

The literature describes three-component films produced from polyelectrolyte complexes formed from commercial citrus peel pectin, chitosan, and polyvinyl alcohol, which exhibited antimicrobial activity against pathogens including *E*. *coli, S. aureus, B. subtilis, Pseudomonas spp.,* and *C*. *albicans* (Tripathi et al., 2010). Kaur and Kaur (Kaur & Kaur, 2012) developed mucoadhesive patches composed of chitosan and pectin for subbuccal application, significantly enhancing the bioavailability of carvedilol compared to an oral formulation intended for swallowing. Also known from the literature are films composed of pectins and itaconic acid polymer for use in contact lenses and dentistry (Teleky & Vodnar, 2019), as well as ion-exchange membranes cross-linked with trivalent ions (Nesic et al., 2011). Films made from high-methylated commercial pectin and chitosan are also known and have been applied as materials for anthocyanin encapsulation, enabling their use as pH indicators in food products (Maciel et al., 2015). Furthermore, Mariniello et al. (Mariniello et al., 2010) developed edible films from phaseolin and pectins isolated from grapefruit albedo and pomelo peel for application as food packaging.

Nevertheless, there remains a search for saccharide-based compounds derived from renewable raw material sources, possessing specific structures that determine their suitability as biomaterials. From Polish patent No. PL 211520 and publications (Pawlaczyk-Graja et al., 2019, 2020), a plant-derived polysaccharide-containing agent, particularly pectin-like, isolated from the leaves of wild strawberry (*Fragaria vesca* L.) with a molecular weight ranging from 4,000 to 1,100,000 Da, is known. The agent comprises polysaccharides in an amount of 10-70% by weight and phenolic compounds associated with the polysaccharide fraction in an amount of 10-50%. The polysaccharide portion of the plant agent consists of residues of glucuronic and galacturonic acids, whose total amount ranges from 20 to 80%, as well as residues of neutral monosaccharides. This agent exhibits several properties, including: (i) as reported in (Szejk, Poplawski, Czubatka-Bienkowska, et al., 2017), the plant agent is non-toxic and does not induce an immune response; (ii) as disclosed in patent No. PL 211520 and scientific publications (Pawlaczyk-Graja et al., 2020; Pawlaczyk et al., 2013), the plant agent exhibits anticoagulant activity, (iii) as disclosed in patent No. PL 216635, antioxidant activity, and (iv) as disclosed in patent No. PL 233306 and publications (Szejk-Arendt et al., 2020; Szejk, Poplawski, Czubatka-Bienkowska, et al., 2017; Szejk, Poplawski, Sarnik, et al., 2017; Zbikowska et al., 2016), protective activity against ionizing radiation.

The essence of the invention is a thin film comprising a plant-derived agent in the form of polysaccharide macromolecules with a molecular weight greater than 4 kDa, obtained from the leaves of wild strawberry *(Fragaria vesca),* having a surface charge density expressed as zeta potential in the range from -50 mV to -5 mV, and consisting of a first layer composed of polyethyleneimine, a second layer composed of calcium alginate, a third layer composed of chitosan, and a fourth layer composed of the plant-derived agent.

A method for producing a thin film comprising a plant-derived agent with a molecular weight greater than 4 kDa, obtained from the leaves of wild strawberry *(Fragaria vesca),* having a surface charge density expressed as a zeta potential in the range from -50 mV to -5 mV, and comprising a first layer of polyethyleneimine, a second layer of calcium alginate, a third layer of chitosan, and a fourth layer of the plant-derived agent, characterized in that a flow deposition method is applied, whereby the first layer of the thin film is formed on a negatively charged solid substrate from polyethyleneimine flowing at a rate of 0.5 ml/min from a 0.001% solution over the solid substrate in a quartz crystal microbalance with dissipation control flow cell until frequency and dissipation signals stabilize, followed by rinsing excess unadsorbed polyelectrolyte with distilled water flowing at 0.5 ml/min over the solid substrate through the quartz crystal microbalance with dissipation control flow cell; the second layer is formed by adsorbing sodium alginate from a 0.2% solution flowing at 0.5 ml/min through the quartz crystal microbalance with dissipation control flow cell over the solid substrate with the first layer until frequency and dissipation signals stabilize, followed by rinsing excess unadsorbed polyelectrolyte with distilled water flowing at 0.5 ml/min over the solid substrate with two layers through the quartz crystal microbalance with dissipation control flow cell; the sodium alginate is crosslinked to calcium alginate by calcium ions from a 0.2 M calcium chloride solution flowing at 0.5 ml/min through the quartz crystal microbalance with dissipation control flow cell over the solid substrate with two layers until frequency and dissipation signals stabilize, followed by rinsing excess salt with distilled water flowing at 0.5 ml/min over the solid substrate with two layers through the quartz crystal microbalance with dissipation control flow cell; the third layer is formed by adsorbing chitosan from a 0.1% solution flowing at 0.5 ml/min through the quartz crystal microbalance with dissipation control flow cell until frequency and dissipation signals stabilize, followed by rinsing excess unadsorbed polyelectrolyte with distilled water flowing at 0.5 ml/min over the solid substrate with three layers through the quartz crystal microbalance with dissipation control flow cell; the fourth layer is formed by adsorbing the plant-derived agent from a solution at a concentration of 0.1% to 5% flowing at 0.5 ml/min through the quartz crystal microbalance with dissipation control flow cell over the solid substrate with three layers until frequency and dissipation signals stabilize, followed by rinsing excess unadsorbed polyelectrolyte with distilled water flowing at 0.5 ml/min over the solid substrate with four layers through the quartz crystal microbalance with dissipation control flow cell.

A method for producing a thin film comprising a plant-derived agent with a molecular weight greater than 4 kDa, obtained from the leaves of wild strawberry *(Fragaria vesca),* having a surface charge density expressed as a zeta potential in the range from -50 mV to -5 mV, and comprising a first layer of polyethyleneimine, a second layer of calcium alginate, a third layer of chitosan, and a fourth layer of the plant-derived agent, characterized in that a flow deposition method is employed, whereby the first layer of the thin film is formed on a negatively charged solid substrate by flowing a 0.001% polyethyleneimine solution at a rate of 0.5 ml/min over the substrate located in a quartz crystal microbalance measurement with dissipation monitoring (QCM-D) cell, until frequency and dissipation signals stabilize; excess unadsorbed polyelectrolyte is rinsed off using distilled water flowing at 0.5 ml/min over the substrate through the quartz crystal microbalance cell; the second layer is formed by adsorption of sodium alginate from a 0.2% solution flowing at 0.5 ml/min through the quartz crystal microbalance cell until frequency and dissipation values stabilize; excess unadsorbed polyelectrolyte is rinsed off with distilled water flowing at 0.5 ml/min over the substrate now bearing one layer; the sodium alginate layer is crosslinked to calcium alginate by exposure to a 0.2 M calcium chloride solution flowing at 0.5 ml/min through the quartz crystal microbalance cell until stabilization of frequency and dissipation signals; excess calcium salt is rinsed off with distilled water flowing at 0.5 ml/min; the third layer is formed by adsorption of chitosan from a 0.1% solution flowing at 0.5 ml/min through the quartz crystal microbalance cell until signal stabilization; excess unadsorbed polyelectrolyte is removed by rinsing with distilled water at 0.5 ml/min; the fourth layer is formed by adsorption of the plant-based agent from a solution with a concentration ranging from 0.1% to 5%, flowing at 0.5 ml/min through the QCM-D cell until frequency and dissipation signals stabilize; excess unadsorbed material is rinsed off with distilled water flowing at 0.5 ml/min.

A method for producing a thin film comprising a plant-derived agent with a molecular weight greater than 4 kDa, obtained from the leaves of wild strawberry *(Fragaria vesca),* having a surface charge density expressed as a zeta potential in the range from -50 mV to -5 mV, and comprising a first layer of polyethyleneimine, a second layer of calcium alginate, a third layer of chitosan, and a fourth layer of the plant-derived agent, characterized in that the first layer is formed by immersing a negatively charged solid substrate in a polyethyleneimine solution at 500 ppm for 10 minutes at room temperature, followed by five successive rinses in distilled water to remove unadsorbed polyelectrolyte; the second layer is formed by spin-coating calcium alginate at 2% concentration at 12,000 rpm for 4 minutes, followed by five successive rinses in distilled water; the sodium alginate layer is crosslinked to calcium alginate by immersing the substrate in a 0.2 M calcium chloride solution for 10 minutes at room temperature, followed by five rinses in distilled water; the third layer is formed by immersing the substrate in a 0.1% chitosan solution for 10 minutes at room temperature, followed by five rinses in distilled water; the fourth layer is formed by immersing the substrate in a solution of the plant-based agent at a concentration of 0.1% to 5% for 10 minutes at room temperature, followed by five rinses in distilled water.

### Example 1

A plant-derived agent (FVU) with a molecular weight greater than 4 kDa, obtained from leaves of wild strawberry *(Fragaria vesca)* by a method according to Polish patent No. PL 211520 and the scientific publication by Pawlaczyk et al. (Pawlaczyk-Graja et al., 2019) via ultrasound-assisted extraction, was applied for the formation of thin films using a dip-coating method. The application of the aforementioned plant-derived agent and the results of tests carried out on the obtained thin film are described below.

The surface charge of the plant-derived agent was determined based on the electrophoretic mobility of FVU particles in water (0.1% w/v) using folded capillary cells with electrodes and expressed as the zeta potential (Zetasizer Nano ZS, Malvern, United Kingdom). A four-layer thin film was constructed on a silicon wafer (10×10 mm). The first layer of the thin film comprised polyethyleneimine (PEI) adsorbed from a 0.001% solution; the second layer consisted of sodium alginate adsorbed from a 0.2% solution and crosslinked with calcium ions at a concentration of 0.2 M (Alg_Ca); the third layer was chitosan (Chi) adsorbed from a 0.1% solution; and the fourth layer comprised FVU adsorbed from a 0.1% solution (10 minutes, 22°C). Residues of non-adsorbed polyelectrolytes were removed by multiple dipping of the silicon wafers with the thin film in distilled water. To determine the thickness of the formed film, individual ellipsometric spectra (EP4, Accurion, Germany) were recorded after the deposition and drying of each successive polyelectrolyte layer.

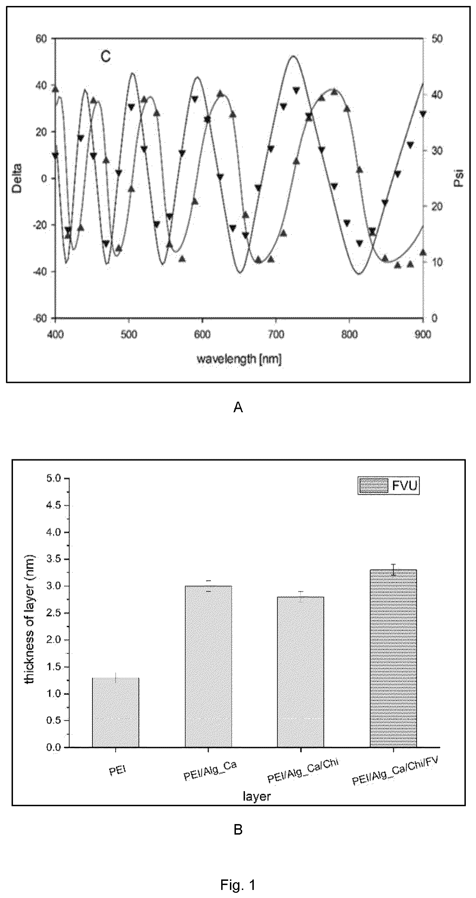

The zeta potential value of FVU (ζ = -38.9 ± 5.0 mV) indicates that it carries a negative surface charge, enabling electrostatic interactions, for example, with a substrate possessing a positive charge of similar magnitude. Fig. 1a shows ellipsometric spectrum of the PEI/Alg_Ca/Chi/FVU film constructed on a silicon wafer. **▲** and **▼** denote experimental spectra, while lines represent the fit of optical models (left). Fig. 1a shows change in the thickness of the thin film formed on the silicon wafer surface with increasing number of polyelectrolyte multilayers (right).

The thickness of the thin film was 3.8 nm, with the thickness of the multilayer formed from FVU being 1.0 nm, confirming the successful deposition of the plant-derived agent as a layer on the flat surface through electrostatic interactions, and consequently the formation of a multilayer thin film.

### Example 2

A plant-derived agent (FVM) with a molecular weight greater than 4 kDa, obtained from leaves of wild strawberry (*Fragaria vesca*) by a method according to Polish patent No. PL 211520 and the scientific publication by Pawlaczyk et al. (Pawlaczyk-Graja et al., 2019) via microwave-assisted extraction, was applied for the formation of thin films using a flow deposition method. The application of the aforementioned plant-derived agent and the results of tests conducted on the obtained thin film are described below.

The surface charge of the plant-derived agent was determined based on the electrophoretic mobility of FVM particles in water (1.0% w/v) using folded capillary cells with electrodes and expressed as the zeta potential (Zetasizer Nano ZS, Malvern, United Kingdom). A four-layer thin film was constructed on a gold-coated piezoelectric quartz sensor. The first layer of the thin film comprised polyethyleneimine (PEI) adsorbed from a 0.001% solution stream flowing through the quartz crystal microbalance with dissipation monitoring (QCM-D) measurement cell (QSense E4, Biolin Scientific, Sweden) at a flow rate of 0.5 mL/min; the second layer consisted of sodium alginate adsorbed from a 0.2% solution flowing through the cell and crosslinked with calcium ions at a concentration of 0.2 M (Alg_Ca); the third layer was chitosan (Chi) adsorbed from a 0.1% solution; and the fourth layer comprised the plant-derived agent (FVM) adsorbed from a 1% solution flowing through the cell (10 minutes, temperature 22°C). Adsorption of the polyelectrolyte molecules was continued until the dissipation signals and frequency shift values stabilized. Unbound molecules were removed by rinsing with distilled water. To determine the thickness, mass, viscosity, and viscoelastic properties of each layer of the thin film, measurements were performed in situ for each layer individually using QSense Dfind software (Biolin Scientific, Sweden).

The zeta potential value of the plant-derived agent FVM (ζ = -38.5 ± 7.0 mV) indicates that it carries a negative surface charge, which enables electrostatic interactions, for example, with a substrate possessing a positive charge of similar magnitude.

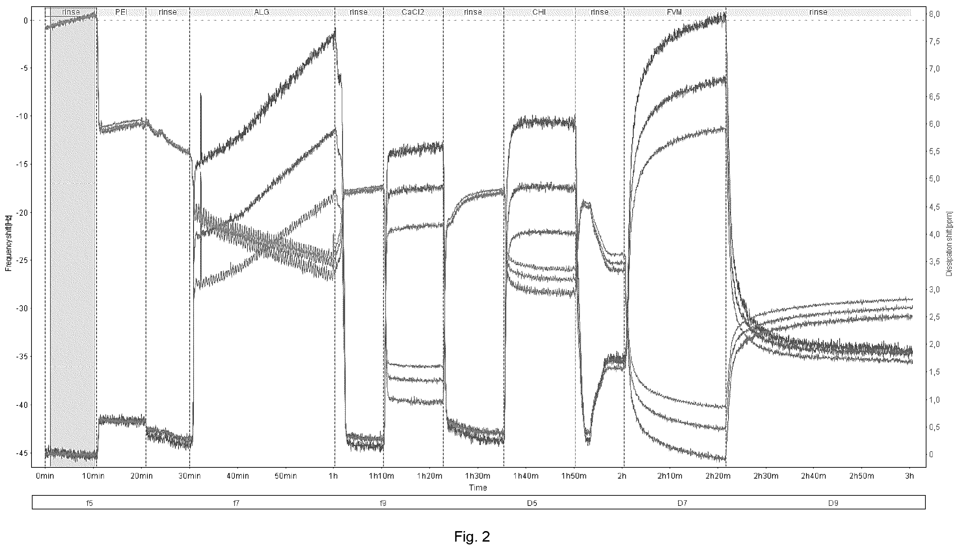

**Fig. 2** shows changes in resonance frequency (grey lines) and dissipation (black lines) values of gold-coated piezoelectric quartz sensors with increasing number of thin film layers.

**Tabela 1. Characteristics of the thin film layers adsorbed onto gold-coated quartz sensors**

| Layer | Surface Mass (ng/cm²) | Viscosity (µPa × s) | Thickness (nm) |
|---|---|---|---|
| **PEI** | 191,6 ± 0,5 | 2725 ± 247 | 4 ± 0.1 |
| **PEI/Alg_Ca** | 322,1 ± 0,4 | 1648 ± 56 | 6 ± 0.3 |
| **PEI/Alg_Ca/Chi** | 452,0 ± 0,4 | 1512 ± 127 | 12 ± 0.4 |
| **PEI/Alg_Ca/Chi/FVM** | 535,2 ± 0,3 | 1651 ± 113 | 15 ± 0.2 |

The thickness of the thin film was 15 nm, with the thickness of the layer formed from FVM being 3 nm, confirming the effective deposition of the plant-derived agent as a layer on a flat surface through electrostatic interactions, and consequently the formation of a multilayer thin film.

In the method according to the invention, the first layer of the thin film is built on a negatively charged solid substrate from polyethyleneimine (PEI), using a 0.001% solution stream flowing at a rate of 0.5 mL/min over the solid substrate in a quartz crystal microbalance with dissipation monitoring (QCM-D) measurement cell, until the frequency shift and dissipation values stabilize. Excess non-adsorbed polyelectrolyte is removed by rinsing with distilled water flowing at a rate of 0.5 mL/min over the solid substrate through the QCM-D measurement cell. The second layer is built from sodium alginate adsorbed from a 0.2% solution flowing at a rate of 0.5 mL/min through the QCM-D measurement cell over the solid substrate with one layer, until the frequency shift and dissipation values stabilize. Excess non-adsorbed polyelectrolyte is removed by rinsing with distilled water flowing at a rate of 0.5 mL/min over the solid substrate with two layers through the QCM-D measurement cell. Sodium alginate is then crosslinked into calcium alginate by means of calcium ions from a 0.2 M calcium chloride solution flowing at a rate of 0.5 mL/min through the QCM-D measurement cell over the solid substrate with two layers, until the frequency shift and dissipation values stabilize. Excess salts are removed by rinsing with distilled water flowing at a rate of 0.5 mL/min over the solid substrate with two layers through the QCM-D measurement cell. The third layer is built from chitosan adsorbed from a 0.1% solution flowing at a rate of 0.5 mL/min through the QCM-D measurement cell over the solid substrate with two layers, until the frequency shift and dissipation values stabilize. Excess non-adsorbed polyelectrolyte is removed by rinsing with distilled water flowing at a rate of 0.5 mL/min over the solid substrate with three layers through the QCM-D measurement cell. The fourth layer is built from the plant-derived agent adsorbed from a solution with a concentration ranging from 0.1% to 5%, flowing at a rate of 0.5 mL/min through the QCM-D measurement cell over the solid substrate with three layers, until the frequency shift and dissipation values stabilize. Excess non-adsorbed polyelectrolyte is removed by rinsing with distilled water flowing at a rate of 0.5 mL/min over the solid substrate with four layers through the QCM-D measurement cell.

### Example 3

A plant-derived agent with a molecular weight greater than 4 kDa, obtained from the leaves of wild strawberry *(Fragaria vesca)* by the method disclosed in Polish patent no. PL 211520 and the scientific publication by Pawlaczyk et al. (Pawlaczyk-Graja et al., 2019) through cold extraction (FVC), was used for the fabrication of thin films employing the dip-coating method. Below is a description of the application procedure of said plant-derived agent and the results of investigations conducted on the obtained thin films.

The surface charge of the plant-derived agent was determined based on the electrophoretic mobility of FVC molecules in water (5.0% w/v) using folded capillary cells with electrodes and expressed as zeta potential (Zetasizer-Nano-ZS, Malvern, United Kingdom). A four-layer thin film was constructed on a silicon wafer (10 x 10 mm). The first layer of the thin film consisted of polyethyleneimine (PEI) adsorbed from a 500 ppm solution, the second layer of sodium alginate deposited by spin coating (50 µl; 12,000 rpm, 4 minutes) (WS-650 Spin Coater, Laurell, USA) and cross-linked with calcium ions (Alg_Ca), the third layer of chitosan (Chi) adsorbed from a 0.4% solution, and the fourth layer of FVC adsorbed from a 5.0% solution (10 minutes, temperature 22°C). Residual, non-adsorbed polyelectrolytes were removed by repeatedly immersing the mica plates with the thin film in distilled water. To determine the thickness of the resulting film, individual ellipsometric spectra (EP4, Accurion, Germany) were recorded after the application and drying of each successive polyelectrolyte layer.

The zeta potential of the plant-derived agent FVC (ζ = -32.9 ± 9.0 mV) indicates that it possesses a negative surface charge, allowing it to electrostatically interact with substrates carrying a positive charge of similar magnitude.

In the method according to the invention, the first layer of the thin film is built on a negatively charged solid substrate from a flow of a 0.001% polyethyleneimine solution, flowing at a rate of 0.5 ml/min over the solid substrate in a quartz crystal microbalance (QCM-D) measurement cell with dissipation monitoring, until the frequency and dissipation signals stabilize. The excess, non-adsorbed polyelectrolyte is then rinsed off using distilled water flowing at 0.5 ml/min over the solid substrate through the QCM-D measurement cell. The second layer is formed from sodium alginate adsorbed from a 0.2% solution flowing at 0.5 ml/min through the QCM-D measurement cell over the solid substrate with one layer, until the frequency and dissipation signals stabilize. Excess, non-adsorbed polyelectrolyte is rinsed off with distilled water flowing at 0.5 ml/min over the solid substrate with two layers through the QCM-D measurement cell. The sodium alginate is then cross-linked to calcium alginate using calcium ions derived from a 0.2M calcium chloride solution flowing at 0.5 ml/min through the QCM-D measurement cell over the solid substrate with two layers, until the frequency and dissipation signals stabilize. Excess salts are removed by rinsing with distilled water flowing at 0.5 ml/min over the solid substrate with two layers through the QCM-D measurement cell. The third layer is formed from chitosan adsorbed from a 0.1% solution flowing at 0.5 ml/min through the QCM-D measurement cell over the solid substrate with two layers, until the frequency and dissipation signals stabilize. Excess, non-adsorbed polyelectrolyte is rinsed off using distilled water flowing at 0.5 ml/min over the solid substrate with three layers through the QCM-D measurement cell. The fourth layer is formed from the plant-derived agent adsorbed from a solution with a concentration ranging from 0.1% to 5%, flowing at a rate of 0.5 ml/min through the QCM-D measurement cell over the solid substrate with three layers, until the frequency and dissipation signals stabilize. Excess, non-adsorbed polyelectrolyte is rinsed off using distilled water flowing at 0.5 ml/min over the solid substrate with four layers through the QCM-D measurement cell.

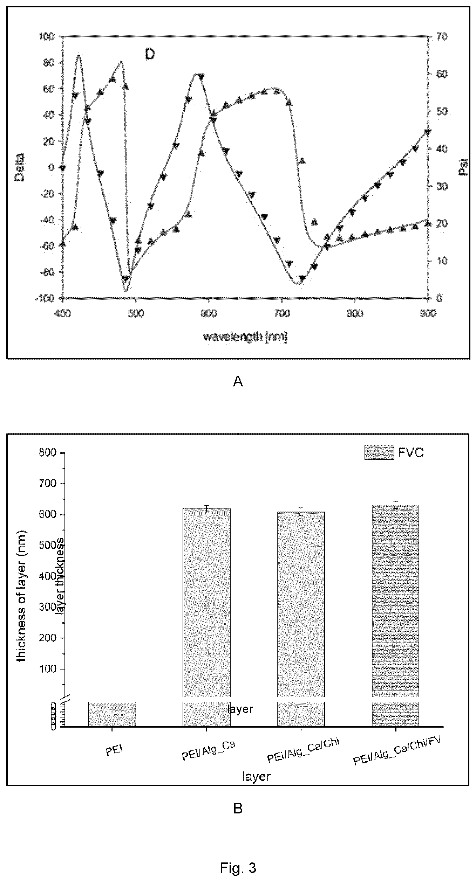

Fig. 3a shows ellipsometric spectrum of the PEI/Alg_Ca/Chi/FVC thin film constructed on a silicon wafer. **▲** and **▼** denote experimental spectra, while the lines represent the fit of optical models (left). Fig. 3b shows change in thickness of the thin film formed on the silicon wafer surface with increasing number of polyelectrolyte multilayers (right).
The applied measurement technique enabled direct determination of the film thickness as the number of polyelectrolyte layers deposited on the substrate increased. The total film thickness was 631 nm, with the layer formed from FVC measuring 22 nm in thickness. This confirms the effective deposition of the plant-derived agent onto the flat surface through electrostatic interactions, resulting in the formation of a multilayer thin film.

## Claims

1. A thin film comprising a plant-based agent in the form of polysaccharide macromolecules having a molecular weight greater than 4 kDa, obtained from leaves of wild strawberry *(Fragaria vesca),* **characterized in that** it has a surface charge density expressed as a zeta potential in the range from -50 mV to -15 mV, and comprises: a first layer of polyethyleneimine, a second layer of calcium alginate, a third layer of chitosan, and a fourth layer of the plant-based agent.

2. A method of producing a thin film comprising a plant-based agent having a molecular weight greater than 4 kDa, obtained from leaves of wild strawberry *(Fragaria vesca),* with a surface charge density expressed as a zeta potential in the range from -50 mV to -15 mV, the thin film comprising: a first layer of polyethyleneimine, a second layer of calcium alginate, a third layer of chitosan, and a fourth layer of the plant-based agent, **characterized in that** the method comprises a flow deposition technique, wherein: (a) the first layer is formed by flowing a 0.001% polyethyleneimine solution at a rate of 0.5 ml/min over a negatively charged solid substrate inside a quartz crystal microbalance with dissipation monitoring (QCM-D) cell until the frequency and dissipation signals stabilize; (b) excess unadsorbed polyethyleneimine is rinsed off by flowing distilled water at 0.5 ml/min over the substrate through the QCM-D cell; (c) the second layer is formed by adsorbing sodium alginate from a 0.2% solution flowing at 0.5 ml/min through the QCM-D cell until frequency and dissipation signals stabilize; (d) excess unadsorbed sodium alginate is rinsed off by flowing distilled water at 0.5 ml/min over the substrate; (e) sodium alginate is crosslinked to calcium alginate by flowing a 0.2 M calcium chloride solution at 0.5 ml/min through the QCM-D cell over the substrate until frequency and dissipation signals stabilize; (f) excess calcium chloride is rinsed off by flowing distilled water at 0.5 ml/min over the substrate; (g) the third layer is formed by adsorbing chitosan from a 0.1% solution flowing at 0.5 ml/min through the QCM-D cell until frequency and dissipation signals stabilize; (h) excess unadsorbed chitosan is rinsed off by flowing distilled water at 0.5 ml/min over the substrate; (i) the fourth layer is formed by adsorbing the plant-based agent from a solution with a concentration between 0.1% and 5% flowing at 0.5 ml/min through the QCM-D cell until frequency and dissipation signals stabilize; (j) excess unadsorbed plant-based agent is rinsed off by flowing distilled water at 0.5 ml/min over the substrate.

3. A method of producing a thin film comprising a plant-based agent having a molecular weight greater than 4 kDa, obtained from leaves of wild strawberry *(Fragaria vesca),* with a surface charge density expressed as a zeta potential in the range from -50 mV to -15 mV, the thin film comprising: a first layer of polyethyleneimine, a second layer of calcium alginate, a third layer of chitosan, and a fourth layer of the plant-based agent, **characterized in that** the method comprises a dipping technique, wherein: (a) the first layer is formed by immersing a negatively charged solid substrate in a 500 ppm polyethyleneimine solution for 10 minutes at room temperature, followed by rinsing off excess unadsorbed polyethyleneimine by five successive immersions in distilled water; (b) the second layer is formed by spin-coating a 2% calcium alginate solution at 12,000 rpm for 4 minutes, followed by rinsing off excess unadsorbed calcium alginate by five successive immersions in distilled water; (c) the sodium alginate layer is crosslinked to calcium alginate by immersing the substrate in a 0.2 M calcium chloride solution for 10 minutes at room temperature, followed by rinsing off excess calcium chloride by five successive immersions in distilled water; (d) the third layer is formed by immersing the substrate in a 0.1% chitosan solution for 10 minutes at room temperature, followed by rinsing off excess unadsorbed chitosan by five successive immersions in distilled water; (e) the fourth layer is formed by immersing the substrate in a solution of the plant-based agent having a concentration between 0.1% and 5% for 10 minutes at room temperature, followed by rinsing off excess unadsorbed agent by five successive immersions in distilled water.
